# EUROPÄISCHE PATENTSCHRIFT

(11) **EP 3 884 546 B1**
(45) Veröffentlichungstag und Bekanntmachung des Hinweises auf die Patenterteilung: **27.09.2023**
(21) Anmeldenummer: 19829402.7
(22) Anmeldetag: 18.11.2019
(51) Int. Cl.: H05K 1/02, H05K 3/36, A61N 1/05, A61N 1/375, H01R 12/78, H01R 13/52

(54) **IMPLANTIERBARE ELEKTRISCHE VERBINDUNGSEINRICHTUNG**
IMPLANTABLE ELECTRICAL CONNECTING DEVICE
AGENCEMENT DE LIAISON ÉLECTRIQUE IMPLANTABLE

(30) Priorität: 21.11.2018 DE 102018219917
(43) Veröffentlichungstag der Anmeldung: 29.09.2021
(73) Patentinhaber: Albert-Ludwigs-Universität Freiburg, 79098 Freiburg (DE)
(72) Erfinder: KIELE, Patrick, 79106 Freiburg (DE); SINGLER, Eva, 79206 Breisach (DE); STIEGLITZ, Thomas, 79110 Freiburg (DE); ORDONEZ, Juan Sebastian, 9000 Gent (BE)
(74) Vertreter: Grünecker Patent- und Rechtsanwälte PartG mbB
(86) Internationale Anmeldenummer: PCT/EP2019/081629
(87) Internationale Veröffentlichungsnummer: WO 2020/104364

(56) Entgegenhaltungen:
- DE-B3-102017 208 625
- US-A1- 2012 149 246
- US-A1- 2016 235 989
- US-B1- 6 368 147

## Beschreibung

Die vorliegende Erfindung betrifft eine implantierbare elektrische Verbindungseinrichtung zum Verbinden einer ersten implantierten Komponente mit einer zweiten implantierten Komponente.

Implantierbare Verbindungseinrichtungen (nachfolgend auch als Konnektoren bezeichnet) spielen eine wichtige Rolle bei der Herstellung und Implantation von aktiven implantierbaren medizinischen Geräten (Active Implantable Medical Device, AIMD). Diese bestehen üblicherweise aus einem Gehäuse, das eine Steuerelektronik und eine Batterie beinhaltet, implantierbaren Elektroden (oder Elektrodenarrays) und Kabeln für die elektrische Kontaktierung der Elektroden mit der Steuerelektronik. Ein Problem hierbei ist, dass zu Beginn der Implantation die einzelnen Komponenten meist nicht fest miteinander verbunden sein dürfen, da sich die Elektroden und die elektrische Einheit in einiger Entfernung zu einander befinden, was zur Folge hätte, dass der gesamte Bereich, indem das Kabel verlaufen würde, aufgeschnitten werden müsste. Ein Beispiel dafür ist die Tiefenhirnstimulation, bei der sich die Elektroden im Gehirn befinden, die Elektronik hingegen im Brustbereich. In diesem Fall werden die Kabel von der Elektronik hin zur Elektrode getunnelt und dort mit Hilfe einer Steckverbindung befestigt. Aktuell limitiert hierbei die Anzahl der Verbindungskontakte im Stecker und dessen Größe die mögliche Anzahl der Elektroden.

Reversibel lösbare Konnektoren ermöglichen es, die elektrisch zu verbindenden Komponenten einzeln zu implantieren, wodurch auch die Möglichkeit geschaffen wird, defekte, verbesserungswürdige oder verbrauchte Teile auszutauschen.

Derartige Implantate und Konnektoren sind z. B. in den Veröffentlichungen J. E. Letechipia, P. H. Peckham, M. Gazdik, and B. Smith, "In-line lead connector for use with implanted neuroprosthesis," IEEE Trans. Biomed. Eng., vol. 38, no. 7, pp. 707-709, 1991; M. Cocco, P. Dario, M. Toro, P. Pastacaldi, and R. Sacchetti, "An implantable neural connector incorporating microfabricated components," J. Micromech. Microeng., vol. 3, no. 4, pp. 219-221, 1993; sowie R. G. Hauser and B. J. Maron, "Lessons from the failure and recall of an implantable cardioverter-defibrillator," (eng), Circulation, vol. 112, no. 13, pp. 2040-2042, 2005; gezeigt.

Die deutsche Patentschrift DE 10 2017 208625 B3 betrifft ein Steckerelement und ein Buchsenelement zum elektrischen Kontaktieren des Steckerelements, insbesondere für den Einsatz bei implantierbaren Komponenten. Das Dokument bezieht sich weiterhin auf entsprechende Herstellungsverfahren zum Herstellen des Steckerelements und des Buchsenelements sowie auf einen implantierbaren miniaturisierten elektrischen Verbinder. Ein Steckerelement umfasst einen elektrisch isolierenden Steckergrundkörper, mindestens einen Kontaktstift, der an einer Kontaktfläche des Steckergrundkörpers so angeordnet ist, dass der Kontaktstift mit einem Buchsenkontakt verbindbar ist, und ein Anschlusselement, das mit dem Kontaktstift elektrisch leitend verbunden ist. Der Kontaktstift weist einen säulenförmigen Vorsprung, der an dem elektrisch isolierenden Steckergrundkörper angeformt ist, eine im Inneren des Vorsprungs verlaufende Leiterbahn, die mit dem Anschlusselement verbunden ist, und eine an einer Außenfläche des Vorsprungs angeordnete Kontaktschicht, die mit der Leiterbahn verbunden und mit dem Buchsenkontakt verbindbar ist, auf.

Das US-Patent US 6 368 147 B1 offenbart einen perkutanen Konnektor ohne Einführungskraft und eine flexible Gehirnsonde. Ein perkutaner Konnektor umfasst eine weibliche Hälfte, die ein Gehäuse mit einem Paar von Seitenwänden umfasst, die jeweils eine Innenfläche aufweisen. Eine elektrische Kontaktanordnung ist entlang der Innenfläche von mindestens einer der Seitenwände angeordnet und hat einen Satz erster elektrischer Kontakte. Isoliermaterial isoliert die elektrischen Kontakte elektrisch voneinander. Darüber hinaus erstreckt sich ein an jedem elektrischen Kontakt befestigter elektrischer Leiter aus dem Gehäuse heraus. Eine männliche Hälfte besteht aus einer Platte aus elastischem Material, die U-förmig gebogen ist und zwei gegenüberliegende Außenflächen hat. Eine Griffanordnung ist so ausgelegt, dass ein Benutzer die beiden gegenüberliegenden Außenflächen näher aneinander drücken kann. Darüber hinaus ist ein Satz zweiter Kontakte an mindestens einer der Außenflächen angebracht und in einer zum ersten Satz von Kontakten passenden Konfiguration angeordnet. Isoliermaterial trennt die Kontakte elektrisch voneinander. Bei dieser Konstruktion kann ein Benutzer die männliche Hälfte an der Griffbaugruppe ergreifen, die beiden Außenflächen zusammendrücken, die männliche Hälfte in die weibliche Hälfte einsetzen und die Griffbaugruppe loslassen, so dass der männliche Satz von Kontakten den weiblichen Satz von Kontakten berührt. Zusätzlich ein Verfahren zur Herstellung einer Elektrodensondenbaugruppe, das die Bereitstellung eines flexiblen Polymersubstrats mit einer Beschichtung aus leitfähigem Material und die Verwendung von Photolithographie und Elektroplattierung zur Bildung eines Satzes von Kontakten und Leitern auf dem flexiblen Polymersubstrat umfasst.

Die Offenlegungsschrift US 2012/149246 A1 bezieht sich auf eine Steckeranordnung und einen Steckverbinder auf der Steckerseite. Auf einer vorderen Oberfläche (einer von zwei gegenüberliegenden Oberflächen) eines steckerseitigen Verbinders sind erste Führungsvorsprünge vorgesehen, die eine Vorsprungshöhe haben, die höher ist als säulenförmige Höcker (steckerseitige Anschlussabschnitte). Auf einer hinteren Oberfläche (der anderen der gegenüberliegenden Oberflächen) eines buchsenseitigen Verbinders sind Lochabschnitte vorgesehen, die eine Größe haben, die eine leichte Bewegung der ersten Führungsvorsprünge erlaubt, wobei die säulenförmigen Erhebungen und die kreuzartigen Schlitze (buchsenseitige Anschlussabschnitte) mit Bezug aufeinander positioniert werden, indem die ersten Führungsvorsprünge durch die Lochabschnitte eingeführt werden.

Die Offenlegungsschrift US 2016/235989 A1 offenbart ein Netzhautprothesensystem mit Nanodraht-Lichtdetektor und Verfahren zu dessen Herstellung. Ein Netzhautprothesensystem umfasst dabei ein flexibles Substrat, einen Nanodraht-Lichtdetektor, der auf dem Substrat angeordnet ist und einen oder mehrere Nanodrähte umfasst, deren Widerstand sich entsprechend dem angelegten Licht ändert, eine oder mehrere Mikroelektroden, die auf dem Substrat angeordnet sind, elektrisch mit dem Nanodraht-Lichtdetektor verbunden sind und in Kontakt mit Netzhautzellen kommen, und eine elektrische Stromversorgungsquelle zum Anlegen von elektrischem Strom an den Nanodraht-Lichtdetektor und die Mikroelektroden. Das Netzhautprothesensystem kann durch die Herstellung eines Nanodraht-Lichtdetektors auf einem Substrat, in das Mikroelektroden eingebaut sind, in ein sehr dünnes und flexibles hochauflösendes Netzhautsystem eingebaut werden.

Je nach gewünschter Anzahl an Kanälen sowie der gewünschten Integrationsdichte nimmt die Größe des Konnektors zu, was wiederum negative Auswirkungen auf das umliegende Gewebe haben kann. Ein Nachteil im Stand der Technik bekannter Verbindungslösungen besteht darin, dass zur verlässlichen Kontaktierung für jeden Kanal separate Federelemente vorgesehen sein müssen, die sich nachteilig auf die gesamte Größe der elektrischen Verbindung auswirken. Zudem wird durch die Federelemente eine relativ große Einbringkraft gefordert, die proportional zur Anzahl der gewünschten elektrischen Kontakte steigt, wodurch die mögliche Anzahl an bereitgestellten Kontakten limitiert wird. Bei miniaturisierten implantierbaren Konnektoren stellt außerdem die elektrische Isolierung zwischen den einzelnen benachbarten Kontakten unterschiedlicher Kanäle ein Problem dar, da durch den Einsatz im Körper eine gesättigte 100%ig feuchte Umgebung vorherrscht. Die elektrische Isolierung muss zusätzlich realisiert werden und benötigt zusätzliche Materialien und Kräfte. Darüber hinaus muss, um eine ausrechende elektrische Isolierung sicherzustellen, bei bekannten Verbinderanordnungen die Distanz zwischen benachbarten Kontakten vergleichsweise groß sein, so dass die Integrationsdichte nicht ausreichend hoch ist. Die Anzahl an Kanälen ist auch aus diesem Grund begrenzt und liegt bei bekannten Anordnungen bei maximal 16.

Der vorliegenden Erfindung liegt die Aufgabe zugrunde, eine implantierbare Verbindungseinrichtung bereitzustellen, die eine sichere elektrische Verbindung implantierter Komponenten bei geringem Platzbedarf und mit einer gegenüber dem Stand der Technik erhöhten Kanaldichte ermöglicht.

Diese Aufgabe durch den Gegenstand der unabhängigen Patentansprüche gelöst. Vorteilhafte Weiterbildungen der vorliegenden Erfindung sind Gegenstand der abhängigen Patentansprüche.

Es wird eine implantierbare elektrische Verbindungseinrichtung zum elektrischen Verbinden einer ersten (implantierbaren/implantierten) Komponente und einer zweiten (implantierbaren/implantierten) Komponente bereitgestellt. Die erste Komponente kann beispielsweise eine Elektrode oder ein Elektrodenarray und die zweite Komponente kann beispielsweise eine Steuerelektronik einer implantierbaren medizinischen Vorrichtung, wie eines Neuorstimulationsgenerators, sein. Die Verbindungseinrichtung umfasst eine erste elastische Mehrlagenschicht mit einer ersten strukturierten elektrisch leitfähigen Schicht und damit elektrisch leitend verbundenen ersten elektrischen Kontakten und eine zweite elastische Mehrlagenschicht mit einer ersten strukturierten elektrisch leitfähigen Schicht und damit elektrisch leitend verbundenen zweiten elektrischen Kontakten, die zur Kontaktierung mit den ersten elektrischen Kontakten der ersten elastischen Mehrlagenschicht ausgebildet sind. Die beiden elastischen Mehrlagenschichten können hierbei insbesondere zu einem Formschluss (der hier nicht notwendig lückenlos sein muss; auch muss sich die Formschlüssigkeit nicht über die gesamten elastischen Mehrlagenschichten erstrecken) miteinander über eine gemeinsame Wicklung der beiden elastischen Mehrlagenschichten ausgebildet sein.

Die erste strukturierte elektrisch leitfähige Schicht und die zweite strukturierte elektrisch leitfähige Schicht können aus einem elektrisch leitfähigen Metall bestehen und weisen durch die Strukturierung mehrere Leiterbahnen (Kanäle) auf, die voneinander elektrisch isoliert sind. Zumindest einige der Kanäle, beispielsweise sämtliche Kanäle, stehen mit jeweils einem oder, zu Redundanzzwecken, mit mehreren der ersten beziehungsweise zweiten elektrischen Kontakten in Verbindung. Andererseits können die Kanäle mit Kontaktpads an Anschlussgebieten der Verbindungseinrichtung zum elektrischen Anschluss an die Komponenten verbunden sein.

Die Eigenschaft der Elastizität der beiden Mehrlagenschichten ist insbesondere dahingehend zu verstehen, dass die Mehrlagenschichten durch eine von menschlicher Hand ausgeübte Krafteinwirkung aufwickelbar sind. Die Verbindungseinrichtung kann durch Aufwickeln der beiden Mehrlagenschichten nach aufeinander Anordnen derselben geschlossen werden, sodass eine mechanisch feste Verbindung mit verlässlicher elektrischer Kontaktierung der einen Mehrlagenschicht mit der anderen erreicht werden kann, ohne dass hierzu irgendwelche Federelemente vonnöten wären. Es wird so eine platzsparende Verbindungseinrichtung bereitgestellt, in der durch die ersten strukturierten elektrisch leitfähigen Schichten eine Vielzahl, insbesondere mehr als 16, Kanäle zur elektrischen Übertragung bereitgestellt werden können.

Die ersten elektrischen Kontakte der ersten elastische Mehrlagenschicht können steckerartige Erhebungen umfassen und die zweiten elektrischen Kontakte der zweiten elastischen Mehrlagenschicht dazu komplementäre buchsenartige Einbuchtungen, wodurch bei gemeinsamer Wickelung der ersten und zweiten elastischen Mehrlagenschicht nach aufeinander Anordnen derselben ein mechanischer Schluss und eine verlässliche elektrische Kontaktierung erreicht werden kann.

Gemäß einer Weiterbildung umfasst die erste elastische Mehrlagenschicht) a) eine erste (elastische) dielektrische Schicht, auf der die erste strukturierte elektrisch leitfähige Schicht der ersten elastischen Mehrlagenschicht ausgebildet ist, b) eine zweite (elastische) dielektrische Schicht, die auf der ersten strukturierten elektrisch leitfähigen Schicht der ersten elastischen Mehrlagenschicht ausgebildet ist, und c) eine zweite elektrisch leitfähige Schicht (beispielsweise aus Metall), wobei der erste elektrische Kontakt der ersten elastischen Mehrlagenschicht die zweite elektrisch leitfähigen Schicht der ersten elastischen Mehrlagenschicht umfasst. Wenn hier und im Weiteren von einer Schicht gesprochen wird, so kann es sich um eine einzelne Schicht oder aber um mehrere Teilschichten handeln. Die erste und die zweite dielektrische Schicht können aus einem Polymermaterial (beispielsweise Polyimid), insbesondere aus dem gleichen Polymermaterial, bestehen oder ein solches umfassen.

Die erste elastische Mehrlagenschicht kann weiterhin eine dritte (elastische) dielektrische Schicht umfassen, auf der die zweite elektrisch leitfähige Schicht der ersten elastischen Mehrlagenschicht teilweise ausgebildet ist. Diese dritte dielektrische Schicht kann ein Polymermaterial (beispielsweise Silikon) umfassen oder daraus bestehen, wobei das Polymermaterial insbesondere ein Elastomer oder ein Gedächtnispolymer umfasst oder daraus besteht. Diese dritte dielektrische Schicht kann zu den oben genannten steckerartigen Erhebungen führen.

In einer weiteren Weiterbildung umfasst die zweite elastische Mehrlagenschicht a) eine erste (elastische) dielektrische Schicht, auf der die erste strukturierte elektrisch leitfähige Schicht der zweiten elastischen Mehrlagenschicht ausgebildet ist, b) eine zweite (elastische) dielektrische Schicht, die auf der ersten strukturierten elektrisch leitfähigen Schicht der zweiten elastischen Mehrlagenschicht ausgebildet ist, und c) eine zweite elektrisch leitfähige Schicht (beispielsweise aus Metall), wobei der zweite elektrische Kontakt der zweiten elastischen Mehrlagenschicht die zweite elektrisch leitfähigen Schicht der zweiten elastischen Mehrlagenschicht umfasst. Auch die erste und die zweite dielektrische Schicht der zweiten elastischen Mehrlagenschicht können aus einem Polymermaterial, insbesondere aus dem gleichen Polymermaterial, bestehen oder ein solches umfassen. Das Polymermaterial kann demjenigen, das für die erste und die zweite dielektrische Schicht der ersten elastischen Mehrlagenschicht Verwendung finden kann, gleich sein.

Die zweite elastische Mehrlagenschicht kann eine dritte (elastische) dielektrische Schicht umfassen, auf der die zweite elektrisch leitfähige Schicht der zweiten elastischen Mehrlagenschicht teilweise ausgebildet ist. Die zweite elektrisch leitfähige Schicht der zweiten elastischen Mehrlagenschicht dient hier also der Ausbildung buchsenartiger elektrischer Kontakte in der dritten dielektrischen Schicht der zweiten elastischen Mehrlagenschicht. Ein mechanischer Schluss kann so mit den steckerartigen Erhebungen der ersten elastischen Mehrlagenschicht bei Aufwicklung der ersten und zweiten elastischen Mehrlagenschicht erreicht werden.

Auch die dritte dielektrische Schicht der zweiten elastischen Mehrlagenschicht kann ein Polymermaterial (beispielsweise Silikon) umfassen oder daraus bestehen, wobei das Polymermaterial insbesondere ein Elastomer oder ein Gedächtnispolymer umfasst oder daraus besteht.

Es wird zudem die implantierbare elektrische Verbindungseinrichtung gemäß einem der oben beschriebenen Beispiele bereitgestellt, in der die erste elastische Mehrlagenschicht und die erste elastische Mehrlagenschicht zumindest teilweise gemeinsam aufgewickelt sind.

Weiterhin wird ein Verfahren zur Herstellung einer implantierbaren einer Verbindungseinrichtung zum elektrischen Verbinden zweier (implantierbarer beziehungsweise implantierter) Komponenten bereitgestellt. Das Verfahren umfasst die Schritte
I) Bilden einer ersten elastischen Mehrlagenschicht, mit
   i.1) Bilden einer ersten dielektrischen Schicht;
   i.2) Bilden einer ersten elektrisch leitfähigen Schicht auf der ersten dielektrischen Schicht;
   i.3) Bilden einer zweiten dielektrischen Schicht auf der ersten elektrisch leitfähigen Schicht;
   i.4) Entfernen eines Teils der zweiten dielektrischen Schicht, um einen Teil der ersten elektrisch leitfähigen Schicht freizulegen; und
   i.5) Bilden von ersten elektrischen Kontakten teilweise über der zweiten dielektrischen Schicht und im elektrischen Kontakt mit der ersten elektrisch leitfähigen Schicht;
      und
II) Bilden einer zweiten elastischen Mehrlagenschicht, mit
   ii.1) Bilden einer weiteren ersten dielektrischen Schicht;
   ii.2) Bilden einer weiteren ersten elektrisch leitfähigen Schicht auf der weiteren ersten dielektrischen Schicht;
   ii.3) Bilden einer weiteren zweiten dielektrischen Schicht auf der weiteren ersten elektrisch leitfähigen Schicht;
   ii.4) Entfernen eines Teils der weiteren zweiten dielektrischen Schicht, um einen Teil der weiteren ersten elektrisch leitfähigen Schicht freizulegen; und
   ii.5) Bilden von zweiten elektrischen Kontakten teilweise über der weiteren zweiten dielektrischen Schicht und im elektrischen Kontakt mit der weiteren ersten elektrisch leitfähigen Schicht.

Hierbei sind die dielektrischen Schichten elastisch, beispielsweise wie oben beschrieben als Polymerschichten, ausgebildet. Dabei kann das Bilden der ersten elektrischen Kontakte das Bilden einer dritten dielektrischen Schicht über der zweiten dielektrischen Schicht der ersten elastischen Mehrlagenschicht und das Bilden einer zweiten elektrisch leitfähigen Schicht teilweise über der dritten dielektrischen Schicht der ersten elastische Mehrlagenschicht und im elektrischen Kontakt mit der ersten elektrisch leitfähigen Schicht der ersten elastische Mehrlagenschicht (10) umfassen, wobei das Verfahren dann weiterhin das Entfernen eines Teils der dritten dielektrischen Schicht, um den Teil der ersten elektrisch leitfähigen Schicht der ersten elastische Mehrlagenschicht freizulegen, umfasst.

Das Bilden der zweiten elektrischen Kontakte kann das Bilden einer dritten dielektrischen Schicht über der zweiten dielektrischen Schicht der zweiten elastischen Mehrlagenschicht, das Entfernen eines Teils der dritten dielektrischen Schicht zum Ausbilden buchsenartiger Einbuchtungen in der zweiten dielektrischen Schicht der zweiten elastischen Mehrlagenschicht und das Bilden einer zweiten elektrisch leitfähigen Schicht an Seitenwänden (und auf dem Boden) der buchsenartiger Einbuchtungen und im elektrischen Kontakt mit der ersten elektrisch leitfähigen Schicht der zweiten elastischen Mehrlagenschicht umfassen.

Das Verfahren gemäß einer der oben genannten Weiterbildungen kann weiterhin das Anordnen der ersten elastischen Mehrlagenschicht und der zweiten elastischen Mehrlagenschicht aufeinander (mit einander zugewandten ersten und zweiten elektrischen Kontakten) und das gemeinsame Aufwickeln der aufeinander angeordneten ersten elastischen Mehrlagenschicht und zweiten elastischen Mehrlagenschicht, sodass eine geschlossene Verbindungseinrichtung mit mechanischem (Form)Schluss und zuverlässiger Kontaktierung der ersten Kontakte mit den zweiten Kontakten entsteht, umfassen.

Weiterhin wird noch bereitgestellt ein Verfahren mit dem Bereitstellen der Verbindungseinrichtung gemäß einer der oben beschriebenen Weiterbildungen, dem Anordnen der ersten elastischen Mehrlagenschicht und der zweiten elastischen Mehrlagenschicht aufeinander (mit einander zugewandten ersten und zweiten elektrischen Kontakten und dem gemeinsamen Aufwickeln der aufeinander angeordneten ersten elastischen Mehrlagenschicht und zweiten elastischen Mehrlagenschicht.

Zum besseren Verständnis der vorliegenden Erfindung wird diese anhand der in den nachfolgenden Figuren dargestellten Ausführungsbeispiele näher erläutert. Dabei werden gleiche Teile mit gleichen Bezugszeichen und gleichen Bauteilbezeichnungen versehen. Weiterhin können auch einige Merkmale oder Merkmalskombinationen aus den gezeigten und beschriebenen unterschiedlichen Ausführungsformen für sich eigenständige, erfinderische oder erfindungsgemäße Lösungen darstellen. Es versteht sich, dass die Ausführungsformen nicht den Bereich der vorliegenden Erfindung erschöpfen.

Dabei zeigen:
- Figur 1: eine schematische Schnittdarstellung einer Verbindungseinrichtung mit zwei Mehrlagenschichten gemäß einer Ausführungsform der vorliegenden Erfindung;
- Figur 2: eine schematische Schnittdarstellung der Verbindungseinrichtung aus Figur 1 nach Anordnung der Mehrlageschichten aufeinander;
- Figur 3: eine Veranschaulichung einer Kontaktverbindung der Verbindungseinrichtung aus Figur 1;
- Figur 4: eine Veranschaulichung einer Verbindungseinrichtung nach Wicklung gemäß einer Ausführungsform der vorliegenden Erfindung;

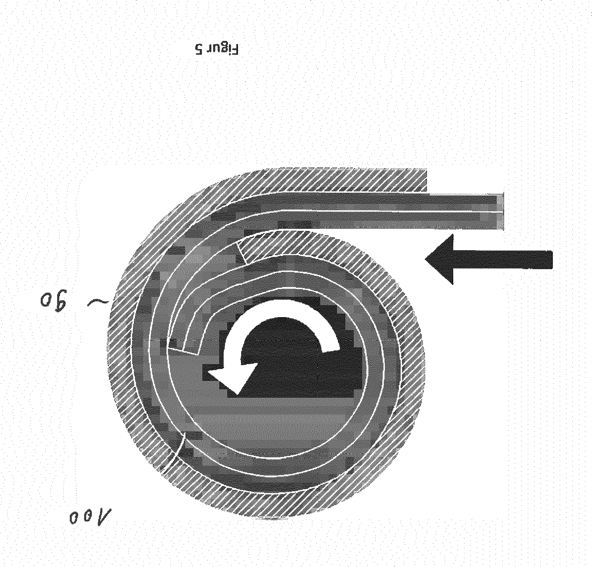

- Figur 5: eine Veranschaulichung eines Fixiermittels zum Fixieren der Verbindungseinrichtung im aufgewickelten Zustand gemäß einer Ausführungsform der vorliegenden Erfindung;
- Figur 6a: eine Veranschaulichung eines Herstellungsprozesses für eine der beiden Mehrlagenschichten der Verbindungseinrichtung gemäß einer Ausführungsform der vorliegenden Erfindung; und
- Figur 6b: eine Veranschaulichung eines Herstellungsprozesses für die andere der beiden Mehrlagenschichten der Verbindungseinrichtung gemäß einer Ausführungsform der vorliegenden Erfindung.

Die vorliegende Erfindung stellt eine Verbindungseinrichtung zum elektrischen Verbinden zweier Komponenten bereit, die in den Körper eines Lebewesen, beispielsweise eines Menschen, implantierbar sind beziehungsweise implantiert worden sind. Bei den Komponenten kann es sich beispielsweise um elektrische Komponenten einer implantierbaren Stimulationseinrichtung handeln. So kann eine der Komponenten ein Gehäuse mit einer Steuerungs- und Auswerteelektronik und einem Stimulator zum Erzeugen und Liefern elektrischer Pulse sein oder umfassen, und es kann die andere Komponente, die mit der erstgenannten elektrisch zu verbinden ist, aus einer oder mehreren Elektroden oder Elektrodenarrays bestehen oder diese umfassen.

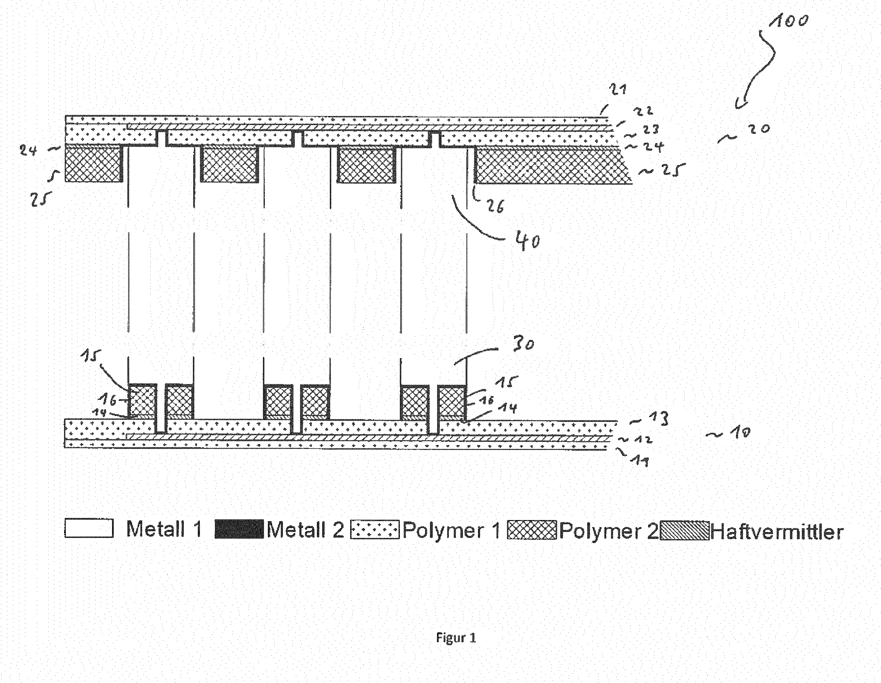

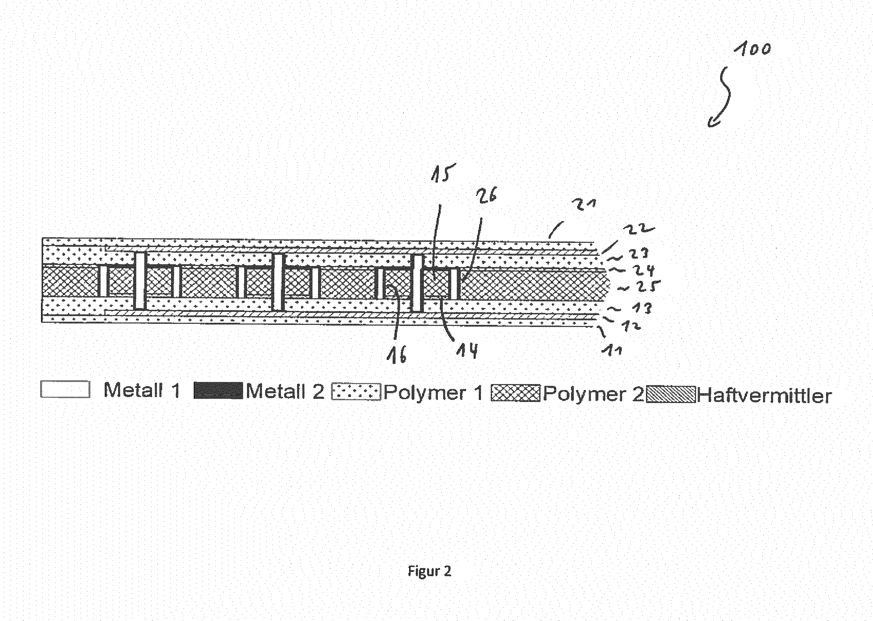

Die Figuren 1 und 2 veranschaulichen eine Verbindungseinrichtung 100 einer erfindungsgemäßen Ausführungsform. Die Verbindungseinrichtung 100 umfasst eine erste elastische Mehrlagenschicht 10 und eine zweite elastische Mehrlagenschicht 20. Die erste Mehrlagenschicht 10 und die zweite Mehrlagenschicht 20 sind für eine Steckverbindung miteinander ausgebildet, wobei die erste Mehrlagenschicht 10 den männlichen Teil der Steckverbindung darstellt, und die zweite Mehrlagenschicht 20 den weiblichen Teil der Steckverbindung darstellt. Die beiden Teile liegen vor einem mechanischen und elektrischen Schluss in planarer Form vor, wie es in den Figuren 1 und 2 gezeigt ist. Sowohl die erste Mehrlagenschicht 10 als auch die zweite Mehrlagenschicht 20 sind dahingehend elastisch ausgebildet, sodass sie ein Aufwickeln unter dem Einfluss von einer von menschlicher Hand aufgebrachten Kraft erlauben (siehe unten).

Die erste Mehrlagenschicht 10 umfasst in dem gezeigten Beispiel eine erste dielektrische (elektrisch isolierende) Schicht 11, eine erste elektrisch leitfähige Schicht 12, eine zweite dielektrische Schicht 13, eine Adhäsionsschicht 14, eine dritte dielektrische Schicht 15 und eine zweite elektrisch leitfähige Schicht 16. Entsprechend umfasst die zweite Mehrlagenschicht 20 in dem gezeigten Beispiel eine erste dielektrische Schicht 21, eine erste elektrisch leitfähige Schicht 22, eine zweite dielektrische Schicht 23, eine Adhäsionsschicht 24, eine dritte dielektrische Schicht 25 und eine zweite elektrisch leitfähige Schicht 26. Die erste elektrisch leitfähige Schicht 12 der ersten Mehrlagenschicht 10 und die erste elektrisch leitfähige Schicht 22 der zweiten Mehrlagenschicht 20 sind strukturiert, sodass sie mehrere Leiterbahnen (Kanäle) bereitstellen. Die zweite elektrisch leitfähige Schicht 16 der ersten Mehrlagenschicht 10 und die zweite elektrisch leitfähige Schicht 26 der zweiten Mehrlagenschicht 20 Stellen Kontaktflächen dar, über die eine elektrische Kontaktierung zwischen der ersten Mehrlagenschicht 10 und der zweiten Mehrlagenschicht 20 im geschlossenen Zustand der Verbindungseinrichtung 100 erfolgt.

Jede Leiterbahn der strukturierten ersten elektrisch leitfähigen Schicht 12 der ersten Mehrlagenschicht 10 und der ersten elektrisch leitfähigen Schicht 22 der zweiten Mehrlagenschicht 20 kann mit einer oder mehreren Kontaktflächen verbunden sein. Die steckerartigen Erhebungen 30, die durch die Schichten 14, 15 und 16 der ersten Mehrlagenschicht 10 gebildet werden, passen in die komplementären Buchsen 40, die durch die Schichten 25 und 26 der zweiten Mehrlagenschicht 20 gebildet werden.

Gemäß einer Ausführungsform können die erste dielektrische Schicht 11 der ersten Mehrlagenschicht 10 und die zweite dielektrische Schicht 13 der ersten Mehrlagenschicht 10 aus einem ersten Polymer bestehen oder ein erstes Polymer umfassen. Alternativ können verschiedene Polymermaterialien für die erste dielektrische Schicht 11 und die zweite dielektrische Schicht 13 verwendet werden. In diesem Fall ist das Vorsehen einer weiteren Adhäsionsschicht zwischen diesen Materialien vorteilhaft. Die erste elektrisch leitfähige Schicht 12 der ersten Mehrlagenschicht 10 kann aus einem ersten Metall bestehen oder ein erstes Metall umfassen, und die zweite elektrisch leitfähige Schicht 16 der ersten Mehrlagenschicht 10 kann aus einem zweiten Metall bestehen oder ein zweites Metall umfassen, das von dem ersten Metall verschieden sein kann. Das erste Metall kann Platin sein, und das zweite Metall kann Gold sein. Die beiden Adhäsionsschichten 14 und 24 können mehrschichtig, beispielsweise aus Siliziumoxid und Siliziumkarbid, aufgebaut sein.

Die dritte dielektrische Schicht 15 der ersten Mehrlagenschicht 10 kann aus einem zweiten Polymer bestehen oder ein zweites Polymer umfassen, wobei das zweite Polymer von dem ersten Polymer verschieden ist. Daher wird die Adhäsionsschicht 14 zur Verbindung der unterschiedlichen Polymermaterialien bereitgestellt. Alternativ kann das zweite Polymer dem ersten gleich sein, sodass auf die Adhäsionsschicht 14 verzichtet werden kann. Gemäß einer weiteren Ausführungsform sind die steckerartigen Erhebungen 30 einteilig aus einem elektrisch leitfähigen (organischen und/oder nichtorganischen) Material oder mehrlagig aus elektrisch leitfähigen (organischen und/oder nichtorganischen) Materialien hergestellt.

Die erste dielektrische Schicht 21 der zweiten Mehrlagenschicht 20 und die zweite dielektrische Schicht 23 der zweiten Mehrlagenschicht 20 können aus einem dritten Polymer bestehen oder ein drittes Polymer umfassen. Alternativ können verschiedene Polymermaterialien für die erste dielektrische Schicht 21 und die zweite dielektrische Schicht 23 verwendet werden. In diesem Fall ist das Vorsehen einer weiteren Adhäsionsschicht zwischen diesen Materialien vorteilhaft. Das dritte Polymer kann dem ersten Polymer, das für die erste Mehrlagenschicht 10 Verwendung findet, gleich sein.

Die erste elektrisch leitfähige Schicht 22 der zweiten Mehrlagenschicht 20 kann aus einem dritten Metall bestehen oder ein drittes Metall umfassen, und die zweite elektrisch leitfähige Schicht 26 der zweiten Mehrlagenschicht 20 kann aus einem vierten Metall bestehen oder ein viertes Metall umfassen, das von dem dritten Metall verschieden sein kann. Das dritte Metall kann dem ersten Metall, das für die erste Mehrlagenschicht 10 Verwendung findet, gleich sein, und das vierte Metall kann dem zweiten Metall, das für die erste Mehrlagenschicht 10 Verwendung findet, gleich sein. Das dritte Metall kann also auch Platin sein, und das vierte Metall kann auch Gold sein. Die dritte dielektrische Schicht 25 der zweiten Mehrlagenschicht 10 kann aus einem vierten Polymer bestehen oder ein viertes Polymer umfassen, wobei das vierte Polymer von dem dritten Polymer verschieden ist. Daher wird die Adhäsionsschicht 24 zur Verbindung der unterschiedlichen Polymermaterialien bereitgestellt. Alternativ kann das vierte Polymer dem dritten gleich sein, sodass auf die Adhäsionsschicht 24 verzichtet werden kann. Das dritte Polymer kann dem ersten Polymer, das für die erste Mehrlagenschicht 10 Verwendung findet, gleich sein, und das vierte Polymer kann dem zweiten Polymer, das für die erste Mehrlagenschicht 10 Verwendung findet, gleich sein.

Zumindest einige der Polymerschichten der ersten Mehrlagenschicht 10 und/oder der zweiten Mehrlagenschicht 20, insbesondere die dritte dielektrische Schicht 25 der zweiten Mehrlagenschicht 20, können aus einem Elastomer, etwa einem Formgedächtnispolymer hergestellt sein. Beispielsweise kann das Elastomer bei Raumtemperatur ein Elastizitätsmodul im Bereich von 10 bis 100 MPa aufweisen. Die erste dielektrische Schicht 11 der ersten Mehrlagenschicht 20 und/oder die zweite dielektrische Schicht 13 der ersten Mehrlagenschicht 10 und/oder die erste dielektrische Schicht 21 der zweiten Mehrlagenschicht 20 und/oder die zweite dielektrische Schicht 23 der zweiten Mehrlagenschicht 20 kann eine Polyimidschicht sein oder eine solche umfassen. Die dritte dielektrische Schicht 15 der ersten Mehrlagenschicht 10 und/oder die dritte dielektrische Schicht 25 der zweiten Mehrlagenschicht 20 kann eine Silikonschicht, beispielsweise eine Polydimethylsiloxan-Schicht, sein oder eine solche umfassen.

Um eine elektrische und mechanische Verbindung zwischen der ersten Mehrlagenschicht 10 und der zweiten Mehrlagenschicht 20 der Verbindungseinrichtung 100 herzustellen, werden diese Mehrlagenschichten zunächst aufeinander angeordnet, wie es in Figur 2 gezeigt ist. In dieser Anordnung können, müssen aber nicht, die Kontaktflächen 16 und 26 bereits in einem elektrischen Kontakt miteinander stehen.

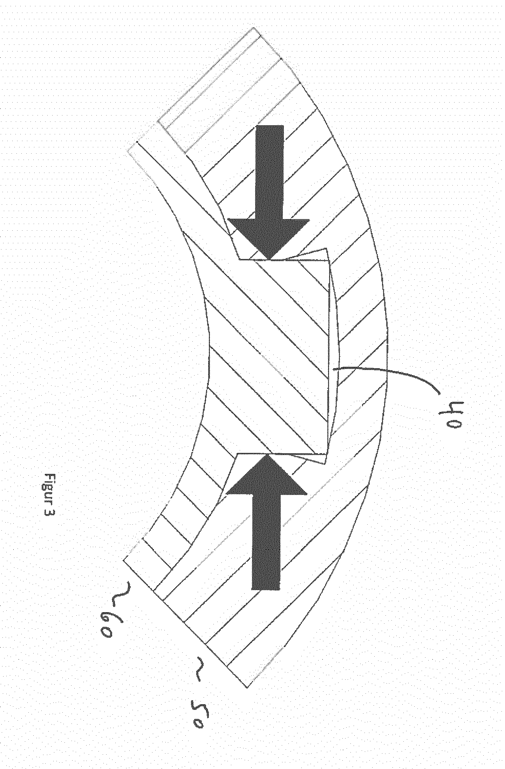

Der mechanische (Form)Schluss (hier nicht notwendig als lückenfrei verstanden) und dadurch die feste elektrische Verbindung zwischen der ersten Mehrlagenschicht 10 und der zweiten Mehrlagenschicht 20 erfolgt durch gemeinsames Aufwickeln der beiden Mehrlagenschichten 10 und 20. Das Aufwickeln kann von menschlicher Hand erfolgen. Die elastischen Mehrlagenschichten 10 und 20 erlauben eine elastische Verformung und der Formschluss zwischen den Mehrlagenschichten 10 und 20 wird durch Querkräfte, die bei der Biegung/Aufwicklung auftreten bewirkt. Durch die Biegung/Aufwicklung wird von den Buchsen 40 der zweiten Mehrlagenschicht 20 an jeder der steckerartigen Erhebungen 30 der ersten Mehrlagenschicht 10 eine Querkraft angelegt, wie es in Figur 3 durch die Pfeile veranschaulicht ist. Der Einfachheit halber zeigt die Figur 3 lediglich eine eine Buchse 40 ausbildende Polymerschicht 50 und eine eine steckerartige Erhebung 30 ausbildende Kontaktschicht 60.

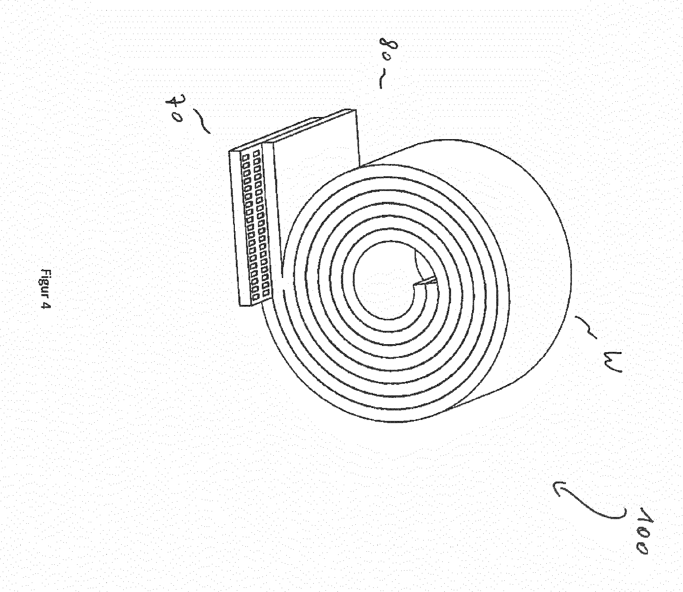

Figur 4 veranschaulicht eine aufgewickelte Verbindungseinrichtung, in der die erste Mehrlagenschicht 10 und die zweite Mehrlagenschicht 20 geschlossen und im elektrischen Kontakt miteinander sind. Mit "aufgewickelt" ist stets vollständig (d.h. im Wesentlichen so weit wie mechanisch ohne Bruch möglich) und teilweise aufgewickelt umfasst. Der Fachmann wird die Wicklung entsprechend der aktuellen Anwendung einzustellen wissen.

Die Wicklung W geht einerseits, elektrisch verbunden mit den Kanälen der strukturierten ersten elektrisch leitfähigen Schicht 22 der zweiten Mehrlagenschicht 20, in ein weibliche Kontaktgebiet 70 und andererseits, elektrisch verbunden mit den Kanälen der strukturierten ersten elektrisch leitfähigen Schicht 12 der ersten Mehrlagenschicht 10, in ein männliches Kontaktgebiet 80 über. Leiterbahnen, die durch die strukturierte erste elektrisch leitfähige Schicht 12 der ersten Mehrlagenschicht 10 ausgebildet sind, sind mit (Löt)Kontaktpads verbunden, die in dem männlichen Kontaktgebiet 80 ausgebildet sind, und Leiterbahnen, die durch die strukturierte erste elektrisch leitfähige Schicht 22 der zweiten Mehrlagenschicht 20 ausgebildet sind, sind mit (Löt)Kontaktpads verbunden, die in dem weiblichen Kontaktgebiet 70 ausgebildet sind. So kann eines der Kontaktgebiete 70 und 80 mit einet elektrischen Komponente und das andere mit einer anderen elektrischen Komponente verbunden werden. Die Verbindung kann beispielsweise über Multi-Wire-Kabel erfolgen. Nach Kontaktierung können die Kontaktpads des weiblichen Kontaktgebiets 70 und des männlichen Kontaktgebiets 80 beispielsweise mithilfe einer Silikonschicht elektrisch isoliert werden. Eines der beiden Kontaktgebiet 70 und 80 kann auch direkt in eine gewünschte Elektrode, beispielsweise eine Detektions- oder Stimulationselektrode übergehen. Weiterhin können auch die beiden Kontaktgebiet 70 und 80, oder zumindest eines davon, zur zusätzlichen Platzersparnis aufgewickelt sein, wodurch aufgrund der sich ergebenden zylinderartigen Form gegebenenfalls auch der elektrische Anschluss an ein Kabel erleichtert werden kann.

Anders als im Stand der Technik erfolgt also keine Steckverbindung über Federelemente, sondern über die beim Aufwickeln wirkenden Kräfte. Durch die Wicklung W kann eine gegenüber dem Stand der Technik signifikante Platzersparnis erreicht werden. Zudem kann gegenüber dem Stand der Technik die Anzahl der zur Verfügung gestellten Kanäle erhöht werden, da keine mit der Anzahl der Kanäle steigende Einbringungskraft für das Herstellen der Verbindung aufgebracht werden muss.

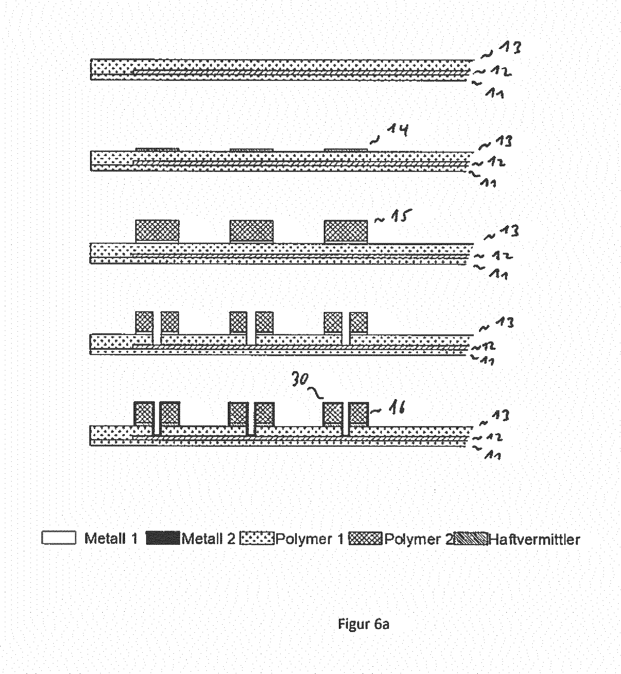

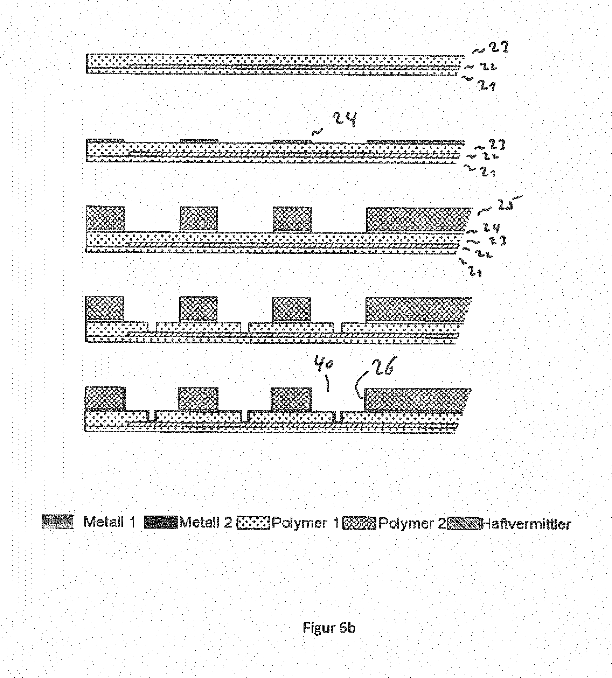

Die in Figur 4 gezeigte Wicklung W kann über ein geeignetes Fixiermittel gegen eine unerwünschte Abwicklung gesichert werden. So kann die fertig aufgewickelte Verbindungseinrichtung mithilfe eines geeignet präparierten Schlauchs, welcher sich beispielsweise bei Erwärmung zusammenzieht, gesichert werden. Der Schlauch kann beispielsweise aus einem Polymermaterial hergestellt werden. Alternativ kann ein geeigneter mechanischer Clip, beispielsweise mit einem darin integrierten Scharnier und einem entsprechenden Verschlussmechanismus, als Fixiermittel bereitgestellt werden. Eine weitere Möglichkeit ist in Figur 6 veranschaulicht. Hierbei ist ein mechanisch stabiles, im Wesentlichen unelastisches und im Inneren spiralförmig ausgebildetes Fixiergehäuse 90 als Fixiermittel vorgesehen, in das hinein die Verbindungseinrichtung 100 durch Einschub in Pfeilrichtung aufgewickelt werden kann.

Die Positionierung der erfindungsgemäßen Verbindungseinrichtung 100 im Körper eines Patienten kann beispielsweise wie folgt erfolgen. Im Auslieferungszustand ist die Verbindungseinrichtung 100 bereits leicht ohne zuverlässigen Formschluss und ohne zuverlässige elektrische Kontaktierung aufgewickelt, um den Querschnitt für das subkutane Tunneln zu verkleinern. Eine Schutzkappe kann die locker aufgewickelte Konfiguration während des subkutanen Tunnelns erhalten und sie vor äußeren Einflüssen schützen. Die Schutzkappe wird nach dem subkutanen Tunneln entfernt, die beiden Mehrlagenschichten werden entrollt, genau aufeinander justiert und nunmehr unter zuverlässigem mechanischen Schluss und zuverlässiger elektrischer Kontaktierung aufgewickelt, und die entstandene Wicklung wird fixiert. Alternativ könnte eine unter zuverlässigem mechanischen Schluss und zuverlässiger elektrischer Kontaktierung aufgewickelte Verbindungseinrichtung 100 subkutan an den Verwendungsort getunnelt werden.

Im Folgenden wird beispielhaft ein Herstellungsverfahren für eine Verbindungseinrichtung, beispielsweise für die in den Figuren 1 und 2 gezeigte Verbindungseinrichtung 100, gemäß einer Ausführungsform der vorliegenden Erfindung anhand der Figuren 6a und 6b beschrieben. Die erste Mehrlagenschicht 10 und die zweite Mehrlagenschicht 20 können unter Verwendung unterschiedlicher Masken mittels eines im Wesentlichen gleichen Schichtprozesses hergestellt werden.

Figur 6a zeigt in der obersten Reihe einen Herstellungszustand für die erste Mehrlagenschicht 10, in dem die erste elektrisch leitfähige Schicht 12 in Form einer Metallschicht auf der ersten dielektrischen Schicht 11 ausgebildet ist. Die erste dielektrische Schicht 11 ist eine Polymerschicht, die beispielsweise durch einen Aufschleuderprozess auf einen geeigneten Träger ausgebildet wird. Die Ausbildung der ersten elektrisch leitfähigen Schicht 12 auf der ersten dielektrischen Schicht 11 erfolgt beispielsweise durch Dampfabscheidung. Nach ihrer Ausbildung wird die erste elektrisch leitfähige Schicht 12 zur Bildung von Leiterbahnen beispielsweise mithilfe eines Laserschneideverfahrens oder durch Photolithographie strukturiert.

Sodann wird die zweite dielektrische Schicht 13, die vorteilhafterweise aus demselben Polymermaterial wie die ersten dielektrischen Schicht 11 hergestellt wird, auf der nach Strukturierung der ersten elektrisch leitfähigen Schicht 12 entstandenen Konfiguration ausgebildet. Auch diese zweite dielektrische Schicht 13 kann durch einen Aufschleuderprozess gebildet werden. Die einzelnen Leiterbahnen der strukturierten ersten elektrisch leitfähigen Schicht 12 werden durch die zweite dielektrische Schicht 13 voneinander elektrisch isoliert. Die beiden Polymerschichten 11 und 13 können Dicken im Bereich von 3 bis 10 µm, beispielsweise 5 µm, aufweisen. Die beiden Polymerschichten 11 und 13 können aus Polyimid bestehen oder diese umfassen. Die Metallschicht 12 kann eine Dicke zwischen etwa 200 und 400 nm, beispielsweise 300 nm, aufweisen. Die Metallschicht 12 kann aus Platin bestehen oder dieses umfassen.

Wie es in der zweiten Reihe von oben in Figur 6a gezeigt ist, wird an geeigneten Stellen eine strukturierte Adhäsionsschicht 14 gebildet. Eine Abscheidung unter Zuhilfenahme einer entsprechenden Abscheidemaske ist zur Ausbildung der strukturierten Adhäsionsschicht 14 möglich. Die Adhäsionsschicht 14 kann eine Teilschicht aus Siliziumdioxid und eine Teilschicht aus Siliziumkarbid umfassen.

Wie es in der mittleren Reihe der Figur 6a gezeigt ist, wird in einem weiteren Herstellungsschritt eine dritte dielektrische Schicht 15 aus einem Polymermaterial, das von demjenigen der ersten und zweiten dielektrischen Schicht 11 und 13 verschieden ist, auf der strukturierte Adhäsionsschicht 14 gebildet. Beispielsweise kann eine flächig abgeschiedene Polymerschicht gebildet werden, aus der dann die nicht gewünschten Bereiche, beispielsweise durch ein Laserschneideverfahren, entfernt werden. Das Polymer der dritten dielektrischen Schicht 15 ist auf der Grundlage der gewünschten Elastizitätseigenschaften hinsichtlich einer festen Verbindung mit den Buchsen 40 der zweiten Mehrlagenschicht (siehe Figuren 1 und 2) auszuwählen. Beispielsweise kann Polydimethylsiloxan oder ein vergleichbares Silikon geeignet gewählt werden.

Die strukturierte erste elektrisch leitfähige Schicht 12 wird zur Kontaktierung durch die strukturierte dritte dielektrische Schicht 15, die Adhäsionsschicht 14 und die erste dielektrische Schicht 11 hindurch freigelegt, wie es in der zweiten Reihe von unten der Figur 6a gezeigt ist. Schließlich wird die zweite elektrisch leitfähige Schicht 16 aus Metall, beispielsweise Gold, in Kontakt mit der strukturierten ersten elektrisch leitfähigen Schicht 12 und auf und an Seitenwänden der dritten dielektrischen Schicht 15 ausgebildet (siehe unterste Reihe der Figur 6a). Die Bildung der zweiten elektrisch leitfähigen Schicht 16 kann mithilfe eines chemischen Dampfabscheidungsverfahrens erfolgen. Die so entstandene Struktur kann sodann zur weiteren Verwendung vom Träger gelöst werden.

Während in dem beschriebenen Beispiel die steckerartigen Erhebungen 30 aus einer Polymer- und einer Metallschicht gebildet werden, können diese alternativ auch aus anderen organischen und/oder anorganischen elektrisch leitfähigen Materialien in Einzel- oder Mehrschichtausführungen gebildet werden.

Die Ausbildung der zweiten Mehrlangenschicht 20 erfolgt ähnlich wie diejenige der ersten Mehrlagenschicht 10. Zunächst wird auf die gleiche Weise der Schichtenstapel, der in der obersten Reihe der Figur 6b gezeigt ist, gebildet. Der Schichtenstapel umfasst die erste dielektrische Schicht 21, die erste elektrisch leitfähige Schicht 22 und die zweite dielektrische Schicht 23. Materialien und Schichtdicken können hierbei wie bei der Ausbildung der ersten Mehrlagenschicht 10 gewählt werden. Sodann wird die strukturierte Adhäsionsschicht 24, etwa auch eine Teilschicht aus Siliziumdioxid und eine Teilschicht aus Siliziumkarbid umfassend, gebildet. Die Strukturierung erfolgt komplementär zu derjenigen der strukturierten Adhäsionsschicht 14 der ersten Mehrlagenschicht 10 (siehe zweite Reihe von oben in Figur 6b). Auf der strukturierten Adhäsionsschicht 24 wird eine weitere Polymerschicht 25 als die dritte dielektrische Schicht 25 gebildet, wie es in der mittleren Reihe der Figur 6b dargestellt ist. Die Wahl der Polymermaterials richtet sich nach den Elastizitätseigenschaften, die für eine stabile Verbindung mit den steckerartigen Erhebungen 30 der ersten Mehrlagenschicht 10 sorgen müssen, sodass bei Biegung/Wicklung auf die schließlich eingebrachten steckerartigen Erhebungen 30 der ersten Mehrlagenschicht 10 eine zum Formschluss hinreichende Querkraft ausgeübt wird (vergleiche Figuren 2 und 3).

Die strukturierte erste elektrisch leitfähige Schicht 22 wird zur Kontaktierung durch die strukturierte dritte dielektrische Schicht 25 und die erste dielektrische Schicht 21 hindurch freigelegt, wie es in der zweiten Reihe von unten der Figur 6b gezeigt ist. Schließlich wird die zweite elektrisch leitfähige Schicht 26 aus Metall, beispielsweise Gold, in Kontakt mit der strukturierten ersten elektrisch leitfähigen Schicht 22 und an Seitenwänden der dritten dielektrischen Schicht 25 ausgebildet, sodass buchsenartige elektrische Kontaktstellen 40 zu der strukturierten ersten elektrisch leitfähigen Schicht 22 entstehen (siehe unterste Reihe der Figur 6b), die im geschlossenen Zustand der Verbindungseinrichtung 100 (siehe Figur 3) die steckerartigen Erhebungen 30 der ersten Mehrlagenschicht 10 kontaktieren.

## Patentansprüche

1. Implantierbare elektrische Verbindungseinrichtung (100) zum elektrischen Verbinden einer ersten und einer zweiten Komponente, wobei die Verbindungseinrichtung (100) umfasst:
eine erste elastische Mehrlagenschicht (10) mit einer ersten strukturierten elektrisch leitfähigen Schicht (12) und damit elektrisch leitend verbundenen ersten elektrischen Kontakten; und
eine zweite elastische Mehrlagenschicht (20) mit einer ersten strukturierten elektrisch leitfähigen Schicht (22) und damit elektrisch leitend verbundenen zweiten elektrischen Kontakten, die zur Kontaktierung mit den ersten elektrischen Kontakten ausgebildet sind,
wobei die ersten elektrischen Kontakte der ersten elastischen Mehrlagenschicht (10) steckerartige Erhebungen (30) umfassen und die zweiten elektrischen Kontakte der zweiten elastischen Mehrlagenschicht (20) buchsenartige Einbuchtungen (40) umfassen, und
die erste elastische Mehrlagenschicht (10)
a) eine erste dielektrische Schicht (11), auf der die erste strukturierte elektrisch leitfähige Schicht (12) der ersten elastischen Mehrlagenschicht (10) ausgebildet ist,
b) eine zweite dielektrische Schicht (13), die auf der ersten strukturierten elektrisch leitfähigen Schicht (12) der ersten elastischen Mehrlagenschicht (10) ausgebildet ist, und
c) eine zweite elektrisch leitfähige Schicht (16) umfasst,
wobei der erste elektrische Kontakt der ersten elastischen Mehrlagenschicht (10) die zweite elektrisch leitfähige Schicht (16) der ersten elastischen Mehrlagenschicht (10) umfasst.

2. Implantierbare elektrische Verbindungseinrichtung (100) gemäß Anspruch 1, in der die erste elastische Mehrlagenschicht (10) und die zweite elastische Mehrlagenschicht (20) zu einem Formschluss miteinander ausgebildet sind.

3. Implantierbare elektrische Verbindungseinrichtung (100) gemäß Anspruch 1, in der die erste elastische Mehrlagenschicht (10) eine dritte dielektrische Schicht (15) umfasst, auf der die zweite elektrisch leitfähige Schicht (16) der ersten elastischen Mehrlagenschicht (10) teilweise ausgebildet ist.

4. Implantierbare elektrische Verbindungseinrichtung (100) gemäß Anspruch 3, in der die dritte dielektrische Schicht (15) der ersten elastischen Mehrlagenschicht (10) ein Polymermaterial umfasst oder daraus besteht, wobei das Polymermaterial insbesondere ein Elastomer oder ein Gedächtnispolymer umfasst oder daraus besteht.

5. Implantierbare elektrische Verbindungseinrichtung (100) gemäß einem der vorhergehenden Ansprüche, in der
die zweite elastische Mehrlagenschicht (20) a) eine erste dielektrische Schicht (21), auf der die erste strukturierte elektrisch leitfähige Schicht (22) der zweiten elastischen Mehrlagenschicht (20) ausgebildet ist, b) eine zweite dielektrische Schicht (23), die auf der ersten strukturierten elektrisch leitfähigen Schicht (22) der zweiten elastischen Mehrlagenschicht (20) ausgebildet ist, und c) eine zweite elektrisch leitfähige Schicht (26) umfasst, wobei der zweite elektrische Kontakt der zweiten elastischen Mehrlagenschicht (20) die zweite elektrisch leitfähigen Schicht (26) der zweiten elastischen Mehrlagenschicht (20) umfasst.

6. Implantierbare elektrische Verbindungseinrichtung (100) gemäß Anspruch 5, in der die zweite elastische Mehrlagenschicht (20) eine dritte dielektrische Schicht (25) umfasst, auf der die zweite elektrisch leitfähige Schicht (26) der zweiten elastischen Mehrlagenschicht (20) teilweise ausgebildet ist.

7. Implantierbare elektrische Verbindungseinrichtung (100) gemäß Anspruch 6, in der die dritte dielektrische Schicht (25) der zweiten elastischen Mehrlagenschicht (20) ein Polymermaterial umfasst oder daraus besteht, wobei das Polymermaterial insbesondere ein Elastomer oder ein Gedächtnispolymer umfasst oder daraus besteht.

8. Implantierbare elektrische Verbindungseinrichtung (100) gemäß einem der vorhergehenden Ansprüche, in der die erste elastische Mehrlagenschicht (10) und die erste elastische Mehrlagenschicht (20) zumindest teilweise gemeinsam aufgewickelt sind.

9. Verfahren zur Herstellung einer implantierbaren Verbindungseinrichtung (100) zum elektrischen Verbinden einer ersten und einer zweiten Komponente, mit den Schritten:
I) Bilden einer ersten elastischen Mehrlagenschicht (10), mit
i.1) Bilden einer ersten dielektrischen Schicht (11);
i.2) Bilden einer ersten elektrisch leitfähigen Schicht (12) auf der ersten dielektrischen Schicht (11);
1.3) Bilden einer zweiten dielektrischen Schicht (13) auf der ersten elektrisch leitfähigen Schicht (12);
i.4) Entfernen eines Teils der zweiten dielektrischen Schicht (13), um einen Teil der ersten elektrisch leitfähigen Schicht (12) freizulegen; und
i.5) Bilden von ersten elektrischen Kontakten teilweise über der zweiten dielektrischen Schicht (13) und im elektrischen Kontakt mit der ersten elektrisch leitfähigen Schicht (12);
und
II) Bilden einer zweiten elastischen Mehrlagenschicht (20), mit
ii.1) Bilden einer weiteren ersten dielektrischen Schicht (21);
ii.2) Bilden einer weiteren ersten elektrisch leitfähigen Schicht (22) auf der weiteren ersten dielektrischen Schicht (13);
ii.3) Bilden einer weiteren zweiten dielektrischen Schicht (13) auf der weiteren ersten elektrisch leitfähigen Schicht (12);
ii.4) Entfernen eines Teils der weiteren zweiten dielektrischen Schicht (13), um einen Teil der weiteren ersten elektrisch leitfähigen Schicht (12) freizulegen; und
ii.5) Bilden von zweiten elektrischen Kontakten teilweise über der weiteren zweiten dielektrischen Schicht (13) und im elektrischen Kontakt mit der weiteren ersten elektrisch leitfähigen Schicht (12),
wobei das Bilden der zweiten elektrischen Kontakte das Bilden einer dritten dielektrischen Schicht (25) über der zweiten dielektrischen Schicht (23) der zweiten elastischen Mehrlagenschicht (20), das Entfernen eines Teils der dritten dielektrischen Schicht (25) zum Ausbilden buchsenartiger Einbuchtungen (40) in der zweiten dielektrischen Schicht (23) der zweiten elastischen Mehrlagenschicht (20) und das Bilden einer zweiten elektrisch leitfähigen Schicht (26) an Seitenwänden der buchsenartigen Einbuchtungen (40) und im elektrischen Kontakt mit der ersten elektrisch leitfähigen Schicht (22) der zweiten elastischen Mehrlagenschicht (20) umfasst.

10. Verfahren gemäß Anspruch 9, in dem
das Bilden der ersten elektrischen Kontakte das Bilden einer dritten dielektrischen Schicht (15) über der zweiten dielektrischen Schicht (13) der ersten elastischen Mehrlagenschicht (10) und das Bilden einer zweiten elektrisch leitfähigen Schicht (16) teilweise über der dritten dielektrischen Schicht (15) der ersten elastischen Mehrlagenschicht (10) und im elektrischen Kontakt mit der ersten elektrisch leitfähigen Schicht (12) der ersten elastischen Mehrlagenschicht (10) umfasst; und
weiterhin mit Entfernen eines Teils der dritten dielektrischen Schicht (15), um den Teil der ersten elektrisch leitfähigen Schicht (12) der ersten elastischen Mehrlagenschicht (10) freizulegen.

11. Verfahren gemäß einem der Ansprüche 9 bis 10, weiterhin mit
Anordnen der ersten elastischen Mehrlagenschicht (10) und der zweiten elastischen Mehrlagenschicht (20) aufeinander; und
gemeinsames Aufwickeln der aufeinander angeordneten ersten elastischen Mehrlagenschicht (10) und zweiten elastischen Mehrlagenschicht (20).

12. Verfahren mit den Schritten:
Bereitstellen der Verbindungseinrichtung (100) gemäß einem der Ansprüche 1 bis 7;
Anordnen der ersten elastischen Mehrlagenschicht (10) und der zweiten elastischen Mehrlagenschicht (20) aufeinander; und
gemeinsames Aufwickeln der aufeinander angeordneten ersten elastischen Mehrlagenschicht (10) und zweiten elastischen Mehrlagenschicht (20).

## Claims

1. An implantable electrical connecting device (100) for electrically connecting a first and a second component, the connecting device (100) comprising:
a first elastic multi-ply layer (10) having a first structured electrically conductive layer (12) and first electrical contacts electrically conductively connected thereto; and
a second elastic multi-ply layer (20) having a first structured electrically conductive layer (22) and second electrical contacts electrically conductively connected thereto and adapted to make contact with the first electrical contacts,
wherein the first electrical contacts of the first elastic multi-ply layer (10) comprise plug-type elevations (30) and the second electrical contacts of the second elastic multi-ply layer (20) comprise socket-type indentations (40), and
the first elastic multi-ply layer (10) comprises
a) a first dielectric layer (11) on which the first structured electrically conductive layer (12) of the first elastic multi-ply layer (10) is formed,
b) a second dielectric layer (13) formed on the first structured electrically conductive layer (12) of the first elastic multi-ply layer (10), and
c) a second electrically conductive layer (16),
wherein the first electrical contact of the first elastic multi-ply layer (10) surrounds the second electrically conductive layer (16) of the first elastic multi-ply layer (10).

2. Implantable electrical connecting device (100) according to claim 1, wherein first elastic multi-ply layer (10) and second elastic multi-ply layer (20) are designed to form a positive engagement with each other.

3. Implantable electrical connecting device (100) according to claim 1, wherein first elastic multi-ply layer (10) comprises a third dielectric layer (15) on which the second electrically conductive layer (16) of the first elastic multilayer layer (10) is partially formed.

4. Implantable electrical connecting device (100) according to claim 3, wherein the third dielectric layer (15) of the first elastic multi-ply layer (10) comprises or consists of a polymer material, the polymer material in particular comprising or consisting of an elastomer or a memory polymer.

5. Implantable electrical connecting device (100) according to any one of the preceding claims, wherein the second elastic multi-ply layer (20) comprises:
a) a first dielectric layer (21) on which the first structured electrically conductive layer (22) of the second elastic multi-ply layer (20) is formed;
b) a second dielectric layer (23) which is formed on the first structured electrically conductive layer (22) of the second elastic multi-ply layer (20), and
c) a second electrically conductive layer (26),
wherein the second electrical contact of the second elastic multi-ply layer (20) comprises the second electrically conductive layer (26) of the second elastic multi-ply layer (20).

6. Implantable electrical connecting device (100) according to claim 5, wherein the second elastic multi-ply layer (20) comprises a third dielectric layer (25) on which the second electrically conductive layer (26) of the second elastic multi-ply layer (20) is partially formed.

7. Implantable electrical connecting device (100) according to claim 6, wherein the third dielectric layer (25) of the second elastic multi-ply layer (20) comprises or consists of a polymer material, the polymer material in particular comprising or consisting of an elastomer or a memory polymer.

8. Implantable electrical connecting device (100) according to any one of the preceding claims, wherein the first elastic multi-ply layer (10) and the first elastic multi-ply layer (20) are at least partially wound up together.

9. A method of manufacturing an implantable connecting device (100) for electrically connecting a first and a second component, comprising the steps of:
I) forming a first elastic multi-ply (10), with
1.1) forming a first dielectric layer (11);
1.2) forming a first electrically conductive layer (12) on the first dielectric layer (11);
1.3) forming a second dielectric layer (13) on the first electrically conductive layer (12);
1.4) removing a portion of the second dielectric layer (13) to expose a portion of the first electrically conductive layer (12); and
1.5) forming first electrical contacts partially over the second dielectric layer (13) and in electrical contact with the first electrically conductive layer (12);
and
II) forming a second elastic multi-ply (20), with
2.1)) forming a further first dielectric layer (21);
2.2)) forming a further first electrically conductive layer (22) on the further first dielectric layer (13);
2.3)) forming a further second dielectric layer (13) on the further first electrically conductive layer (12);
2.4)) removing a portion of the further second dielectric layer (13) to expose a portion of the further first electrically conductive layer (12); and
2.5)) forming second electrical contacts partially over the further second dielectric layer (13) and in electrical contact with the further first electrically conductive layer (12),
wherein the forming of second electrical contacts comprises forming a third dielectric layer (25) over the second dielectric layer (23) of the second elastic multi-ply layer (20), removing a portion of the third dielectric layer (25) for forming socket-type indentations (40) in the second dielectric layer (23) of the second elastic multi-ply layer (20), and forming a second electrically conductive layer (26) at sidewalls of the socket-type indentations (40) and in electrical contact with the first electrically conductive layer (22) of the second elastic multi-ply layer (20).

10. Method according to claim 9, wherein
the forming of the first electrical contacts comprises forming a third dielectric layer (15) over the second dielectric layer (13) of the first elastic multi-ply layer (10) and forming a second electrically conductive layer (16) partially over the third dielectric layer (15) of the first elastic multi-ply layer (10) and in electrical contact with the first electrically conductive layer (12) of the first elastic multi-ply layer (10); and
furthermore with removing a portion of the third dielectric layer (15) to expose the portion of the first electrically conductive layer (12) of the first elastic multi-ply layer (10).

11. Method according to any one of claims 9 to 10, furthermore with
arranging the first elastic multi-ply layer (10) and the second elastic multi-ply layer (20) one upon each other; and
together winding up the first elastic multi-ply layer (10) and second elastic multi-ply layer (20) arranged one upon the other.

12. Method with the steps of:
providing the connecting device (100) according to any one of claims 1 to 7;
arranging the first elastic multi-ply layer (10) and the second elastic multi-ply layer (20) one upon the other; and
together winding up the first elastic multi-ply layer (10) and second elastic multi-ply layer (20) arranged one upon the other.

## Revendications

1. Dispositif de connexion électrique implantable (100) pour connecter électriquement un premier composant et un deuxième composant, dans lequel le dispositif de connexion (100) comprend :
une première multicouche élastique (10) avec une première couche électroconductrice structurée (12) et des premiers contacts électriques qui lui sont connectés électriquement ; et
une deuxième multicouche élastique (20) avec une première couche électroconductrice structurée (22) et des deuxièmes contacts électriques qui lui sont connectés électriquement, conformés pour être en contact avec les premiers contacts électriques,
dans lequel les premiers contacts électriques de la première multicouche élastique (10) comprennent des protubérances en forme de fiche (30) et les deuxièmes contacts électriques de la deuxième multicouche élastique (20) comprennent des renfoncements en forme de prise (40), et
la première multicouche élastique (10) comprend
a) une première couche diélectrique (11) sur laquelle est formée la première couche électroconductrice structurée (12) de la première multicouche élastique (10),
b) une deuxième couche diélectrique (13) sur laquelle est formée la première couche électroconductrice structurée (12) de la première multicouche élastique (10), et
c) une deuxième couche électroconductrice (16),
dans lequel le premier contact électrique de la première multicouche élastique (10) comprend la deuxième couche électroconductrice (16) de la première multicouche élastique (10) .

2. Dispositif de connexion électrique implantable (100) selon la revendication 1, dans lequel la première multicouche élastique (10) et la deuxième multicouche élastique (20) sont conformées pour s'accoupler par complémentarité de forme.

3. Dispositif de connexion électrique implantable (100) selon la revendication 1, dans lequel la première multicouche élastique (10) comporte une troisième couche diélectrique (15) sur laquelle la deuxième couche électroconductrice (16) de la première multicouche élastique (10) est partiellement formée.

4. Dispositif de connexion électrique implantable (100) selon la revendication 3, dans lequel la troisième couche diélectrique (15) de la première multicouche élastique (10) comprend un matériau polymère ou est constituée d'un tel matériau, dans lequel le matériau polymère comprend en particulier un élastomère ou un polymère à mémoire de forme, ou est constitué d'un tel matériau.

5. Dispositif de connexion électrique implantable (100) selon l'une des revendications précédentes, dans lequel
la deuxième multicouche élastique (20) comprend a) une première couche diélectrique (21) sur laquelle est formée la première couche électroconductrice structurée (22) de la deuxième multicouche élastique (20), b) une deuxième couche diélectrique (23) sur laquelle est formée la première couche électroconductrice structurée (22) de la deuxième multicouche élastique (20), et c) une deuxième couche électroconductrice (26), dans lequel le deuxième contact électrique de la deuxième multicouche élastique (20) comprend la deuxième couche électroconductrice (26) de la deuxième multicouche élastique (20).

6. Dispositif de connexion électrique implantable (100) selon la revendication 5, dans lequel la deuxième multicouche élastique (20) comprend une troisième couche diélectrique (25) sur laquelle est partiellement formée la deuxième couche électroconductrice (26) de la deuxième multicouche élastique (20) .

7. Dispositif de connexion électrique implantable (100) selon la revendication 6, dans lequel la troisième couche diélectrique (25) de la deuxième multicouche élastique (20) comprend un matériau polymère ou est constituée d'un tel matériau, dans lequel le matériau polymère comprend en particulier un élastomère ou un polymère à mémoire de forme, ou est constitué d'un tel matériau.

8. Dispositif de connexion électrique implantable (100) selon l'une des revendications précédentes, dans lequel la première multicouche élastique (10) et la deuxième multicouche élastique (20) sont au moins partiellement enroulées conjointement.

9. Procédé de fabrication d'un dispositif de connexion implantable (100) pour connecter électriquement un premier composant et un deuxième composant, comprenant les étapes suivantes :
I) formation d'une première multicouche élastique (10), avec
i.1) la formation d'une première couche diélectrique (11) ;
i.2) la formation d'une première couche électroconductrice (12) sur la première couche diélectrique (11) ;
i.3) la formation d'une deuxième couche diélectrique (13) sur la première couche électroconductrice (12) ;
i.4) l'élimination d'une partie de la deuxième couche diélectrique (13) pour exposer une partie de la première couche électroconductrice (12) ; et
i.5) la formation de premiers contacts électriques partiellement sur la deuxième couche diélectrique (13) et en contact électrique avec la première couche électroconductrice (12) ;
et
II) la formation d'une deuxième multicouche élastique (20), avec
ii.1) la formation d'une autre première couche diélectrique (21) ;
ii.2) la formation d'une autre première couche électroconductrice (22) sur ladite autre première couche diélectrique (13) ;
ii.3) la formation d'une autre deuxième couche diélectrique (13) sur ladite autre première couche électroconductrice (12) ;
ii.4) l'élimination d'une partie de ladite autre deuxième couche diélectrique (13) pour exposer une partie de ladite autre première couche électroconductrice (12) ; et
ii.5) la formation de deuxièmes contacts électriques partiellement sur ladite autre deuxième couche diélectrique (13) et en contact électrique avec ladite autre première couche électroconductrice (12),
dans lequel la formation des deuxièmes contacts électriques comprend la formation d'une troisième couche diélectrique (25) sur la deuxième couche diélectrique (23) de la deuxième multicouche élastique (20), l'élimination d'une partie de la troisième couche diélectrique (25) pour former des renfoncements en forme de prise (40) dans la deuxième couche diélectrique (23) de la deuxième multicouche élastique (20), et la formation d'une deuxième couche électroconductrice (26) sur les parois latérales des renfoncements en forme de prise (40) et en contact électrique avec la première couche électroconductrice (22) de la deuxième multicouche élastique (20).

10. Procédé selon la revendication 9, comprenant
la formation des premiers contacts électriques, la formation d'une troisième couche diélectrique (15) sur la deuxième couche diélectrique (13) de la première multicouche élastique (10), et la formation d'une deuxième couche électroconductrice (16) partiellement sur la troisième couche diélectrique (15) de la première multicouche élastique (10) et en contact électrique avec la première couche électroconductrice (12) de la première multicouche élastique (10) ; et
comprenant en outre l'élimination d'une partie de la troisième couche diélectrique (15) pour exposer ladite partie de la première couche électroconductrice (12) de la première multicouche élastique (10).

11. Procédé selon l'une des revendications 9 à 10, comprenant en outre
l'empilement de la première multicouche élastique (10) et de la deuxième multicouche élastique (20) ; et
l'enroulement conjoint de la première multicouche élastique (10) et de la deuxième multicouche élastique (20) empilées l'une sur l'autre.

12. Procédure comprenant les étapes suivantes :
fourniture du dispositif de connexion (100) selon l'une des revendications 1 à 7 ;
empilement de la première multicouche élastique (10) et de la deuxième multicouche élastique (20) ; et
enroulement conjoint de la première multicouche élastique (10) et de la deuxième multicouche élastique (20) empilées l'une sur l'autre.
